(19) Europäisches Patentamt
European Patent Office
Office européen des brevets

(11) **EP 2 559 382 B1**

(12) **EUROPEAN PATENT SPECIFICATION**

(45) Date of publication and mention
of the grant of the patent:
**07.01.2015 Bulletin 2015/02**

(51) Int Cl.:
**A61B 6/00** *(2006.01)*    **G06F 19/00** *(2011.01)*

(21) Application number: **12177224.8**

(22) Date of filing: **20.07.2012**

(54) **Radiation dose information sharing device and method**

Vorrichtung und Verfahren zum Teilen der Strahlungsdosierungsinformation

Dispositif de partage d'informations de dosage de rayonnement et procédé

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.08.2011 JP 2011177869**

(43) Date of publication of application:
**20.02.2013 Bulletin 2013/08**

(73) Proprietor: **Fujifilm Corporation
Minato-ku
Tokyo (JP)**

(72) Inventor: **Kuwabara, Takeshi
Kanagawa (JP)**

(74) Representative: **Klunker . Schmitt-Nilson . Hirsch
Patentanwälte
Destouchesstraße 68
80796 München (DE)**

(56) References cited:
**EP-A1- 1 410 761      EP-A2- 1 354 554
WO-A1-2011/048547**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

1. Field of the Invention

The present invention relates to a radiation dose information sharing device and method for sharing information about an optimal radiation dose between a plurality of radiation imaging systems.

2. Description Related to the Prior Art

In medical diagnosis and treatment, a radiation imaging system, for example, an X-ray imaging system is widely known. The X-ray imaging system is constituted of an X-ray source for emitting X-rays to a patient's body and an electronic cassette for detecting an X-ray image of the body. The electronic cassette is composed of a flat panel detector (FPD) and a flat rectangular housing containing the FPD. The FPD has a matrix of pixels each of which accumulates signal charge by an amount corresponding to the amount of the X-rays incident thereon. The FPD accumulates the signal charge on a pixel-by-pixel basis, and converts the accumulated signal charge into a voltage signal in its signal processing circuit. Thereby, the FPD electrically detects the X-ray image, and outputs the X-ray image as digital image data. The FPD has X-ray sensitivity much higher than that of an X-ray film and an imaging plate (IP). This allows the FPD to obtain the high-quality X-ray image with a low radiation dose.

The electronic cassette is mounted not only on a specific imaging support, but also on an existing imaging support shareable between a film cassette and an IP cassette. Furthermore, the electronic cassette can be used while being put on a bed or held by the patient himself/herself, to take a radiograph of a body part that is hard to take with a stationary detector. The electronic cassette is sometimes brought out from a hospital for use in bedside radiography of a home-care patient or in an outside accident or natural disaster site in an emergency.

Conventionally, many techniques are proposed to reduce a radiation dose to be applied to the patient. For example, according to Japanese Patent Laid-Open Publication No. 2010-179155, a moving image capturing condition is determined such that the sum total of radiation doses applied during capturing a moving image becomes equal to or less than a radiation dose required for capturing a single static image.

By the way, in a case where a hospital is prepared with a plurality of X-ray imaging systems having different specifications, image quality variations, especially graininess variations among the X-ray images ascribed to the difference between the systems interfere with smooth and accurate diagnosis. For this reason, with the aim of making the image quality uniform, it is preferable to draw up guidelines for optimal doses on the basis of a body part to be imaged, an imaging direction, an imaging position, and the like, and share the guidelines among the plurality of systems. Drawing up the guidelines requires a number of radiography experiences in past, and is difficult for a small hospital having a limited number of patients. Therefore, the small hospital preferably uses the guidelines that a large hospital has drawn up.

However, various problems arise, if the guidelines that have been drawn up based on the X-ray images captured by a sample X-ray imaging system are applied as-is to another type of X-ray imaging system having different specifications. The X-ray sensitivity of the electronic cassette differs from maker to maker and from type to type. Even if the same dose of X-rays is applied, the image quality of the obtained X-ray image varies depending on the sensitivity of the electronic cassette. Furthermore, in the case of overlaying an intermediate member such as a grid on the electronic cassette, the grid absorbs the X-rays by specific absorptance. Accordingly, it is necessary to optimize the guidelines on a system-by-system basis.

By the optimization of the guidelines, a radiation dose to be applied can be sometimes set lower than an original dose set in the sample system. This is not limited to the case of sharing the guidelines of the optimal radiation doses among the plurality of X-ray imaging systems, but the same goes for the case of exchanging a part of the X-ray imaging system because of the deterioration or breakage of the part.

EP 1 410 761 A1 discloses a medical information registering system using examination protocols (including set values for e. g. an X-ray photographing system). The examination protocol is transmitted to a server and upon request the examination protocol may be downloaded so as to be used by an operator for the same X-ray apparatus or a similar X-ray apparatus. The document mentions that the controller of an X-ray apparatus has a function of customising a photographing protocol to the actually used apparatus, e. g. customising the settings of a part of functions of the apparatus such as the position of the supporting device or X-ray exposing conditions.

SUMMARY OF THE INVENTION

An object of the present invention is to provide a radiation dose information sharing device that can easily share information about an optimal radiation dose between radiation imaging systems having different specifications, and a method thereof.

To achieve the above and other objects of the present invention, a radiation dose information sharing device according to the present invention includes. The features of claim 1.

Preferred embodiments are defined by the dependent claims.

A radiation dose information sharing method includes the steps defined in claim 13.

According to the present invention, the first optimal dose set up by the first radiation imaging system is converted into the second optimal dose to be used in the second radiation imaging system based on the first and second specification data of the first and second radiation imaging systems. Therefore, it is possible to easily share radiation dose information between the first and second radiation imaging systems having different specifications.

BRIEF DESCRIPTION OF THE DRAWINGS

For more complete understanding of the present invention, and the advantage thereof, reference is now made to the following descriptions taken in conjunction with the accompanying drawings, in which:

Fig. 1 is a schematic view showing the structure of a first X-ray imaging system installed in a hospital A and a second X-ray imaging system installed in a hospital B;
Fig. 2 is a block diagram of a console;
Fig. 3 is an explanatory view of first specification data;
Fig. 4 is a block diagram showing the function of the console of the second X-ray imaging system in the hospital B and an information flow;
Fig. 5 is an explanatory view of absorptance data;
Fig. 6 is an explanatory view of a data entry window;
Fig. 7 is an explanatory view showing the contents of conversion processing of an arithmetic section;
Fig. 8 is an explanatory view of a conversion result display window;
Fig. 9 is a flowchart showing an operation flow from obtainment of the first specification data to display of a conversion result;
Fig. 10 is a block diagram showing the function of a console of a second embodiment having a day counter and deterioration correction data and an information flow; and
Fig. 11 is an explanatory view of conversion processing of an optimal dose according to a third embodiment in which the specifications of tube voltage differs between the first and second X-ray imaging systems.

DESCRIPTION OF THE PREFERRED EMBODIMENTS

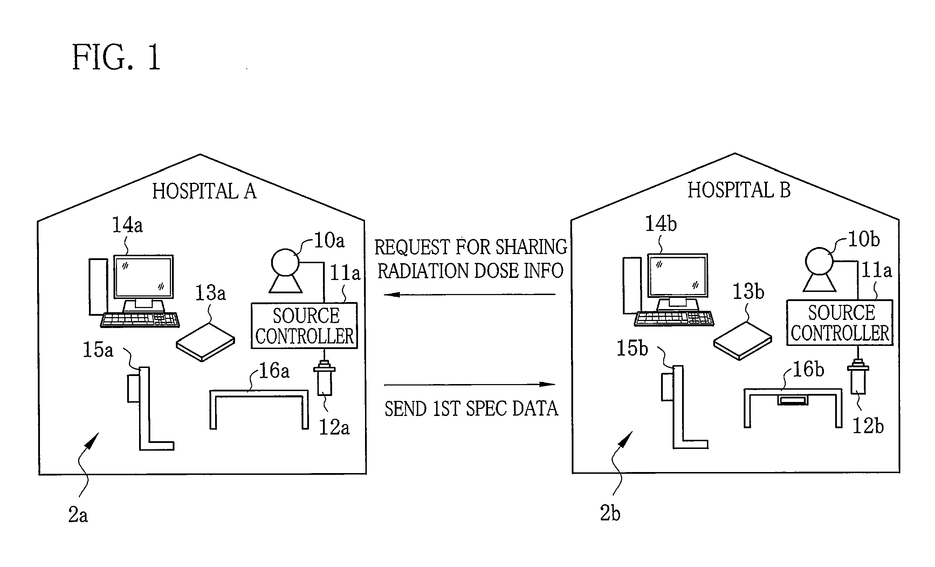

As shown in Fig. 1, a first X-ray imaging system 2a is installed in a hospital A, and a second X-ray imaging system 2b is installed in a hospital B. The first and second X-ray imaging systems 2a and 2b have different specifications from each other, while have almost the same basic configuration. Hereafter, the first X-ray imaging system 2a of the hospital A is described. As for the second X-ray imaging system 2b, components similar to those of the first X-ray imaging system 2a are indicated with the same reference numerals but with a suffix "b" instead of "a", and the description thereof is omitted.

The X-ray imaging system 2a is constituted of an X-ray source 10a, a source controller 11a, an exposure switch 12a, an electronic cassette 13a, a console 14a, an imaging stand 15a, and an imaging table 16a. The X-ray source 10a contains an X-ray tube for emitting X-rays. The source controller 11a controls the operation of the X-ray source 10a. The exposure switch 12a commands the start of X-ray emission. The electronic cassette 13a detects an X-ray image based on the X-rays passed through a patient's body. The console 14a performs the operation control of the electronic cassette 13a and image processing of the X-ray image. The imaging stand 15a is used for taking a radiograph of the patient in a standing position. The imaging table 16a is used for taking a radiograph of the patient in a lying position. Additionally, the first X-ray imaging system 2a is provided with a source moving unit (not shown) for setting the X-ray source 10a in a desired direction and position.

As is widely known, the X-ray source 10a has the X-ray tube for emitting the X-rays, and a collimator for limiting an irradiation field of the X-rays. The X-ray tube has a cathode being a filament for emitting thermoelectrons, and an anode (target) for radiating the X-rays by collision of the thermoelectrons emitted from the cathode. The collimator is composed of four X-ray shielding lead plates disposed on each side of a rectangle so as to form an irradiation opening in its middle through which the X-rays propagate. Changing the positions of the lead plates can vary the size of the irradiation opening to limit the irradiation field.

The source controller 11a includes a high voltage generator and a controller. The high voltage generator supplies high voltage to the X-ray source 10a. The controller controls a tube voltage for determining an energy spectrum of the X-rays from the X-ray source 10a, a tube current for determining an X-ray irradiation amount per unit of time, and an X-ray irradiation duration. The high voltage generator produces the high tube voltage by multiplying an input voltage using a transformer, and supplies drive power to the X-ray source 10a through a high voltage cable. An imaging condition including the tube voltage, the tube current, and the X-ray irradiation duration is set up manually by a radiographic technician from an operation panel of the source controller 11a or from the console 14a through a communication cable.

The exposure switch 12a is a two-step switch operated by the radiographic technician. Upon a half press of the exposure switch 12a, a warm-up start signal is issued to start warming up the X-ray source 10a. Upon a full press of the exposure switch 12a, an irradiation start signal is issued to make the X-ray source 10a start emitting the X-rays. These signals are inputted to the source controller 11a through a signal cable.

The source controller 11a controls the operation of the X-ray source 10a based on the signals from the exposure switch 12a. Upon reception of the irradiation start signal from the exposure switch 12a, the source controller 11a starts supplying electric power to the X-ray source 10a, and, at the same time, actuates a timer to start measuring the X-ray irradiation duration. After a lapse of the predetermined X-ray irradiation duration set up by the imaging condition, the source controller 11a stops application of the X-rays. In taking a static image, a maximum X-ray irradiation duration is set at the order of approximately 500 msec to 3 sec.

As is widely known, the electronic cassette 13a includes a flat panel detector (FPD) and a portable housing containing the FPD. The FPD is of a TFT type based on amorphous silicon or a CMOS type based on single crystal silicon. The FPD has a matrix of pixels each of which accumulates signal charge by an amount corresponding to the amount of X-rays incident thereon. There are two types of FPDs, that is, a direct conversion type for directly converting the X-rays into the signal charge, and an indirect conversion type for converting visible light produced in a scintillator into the signal charge. The FPD accumulates the signal charge on a pixel-by-pixel basis, and converts the accumulated signal charge into a voltage signal at its signal processing circuit. Thereby, the FPD electrically detects the X-ray image, and outputs the X-ray image as digital image data.

The housing of the electronic cassette 13a is in a rectangular and flat box shape and approximately the same size as those of a film cassette and an IP cassette (also called CR cassette). In other words, the electronic cassette 13a is compatible with International Standard ISO4090:2001 in size and shape. Thus, the electronic cassette 13a can be set in an existing imaging support shareable between the film cassette and the IP cassette. A plurality of electronic cassettes 13a are provided in each examination room, for example, one electronic cassette 13a for each of the imaging stand 15a and the imaging table 16a. The electronic cassette 13a is detachably set on the imaging stand 15a or the imaging table 16a in a position such that an imaging plane of the FPD is opposed to the X-ray source 10a. In addition, the electronic cassette 13a is sometimes used separately from the imaging stand 15a or the imaging table 16a in a state of being put on a bed under the patient's body or held by the patient himself/herself.

The electronic cassette 13a is of a wireless type, for example, and establishes wireless communication with the console 14a using a radio wave or infrared light, besides wired communication using a communication cable. The electronic cassette 13a is energized not only from utility power through a power cable, but also from an internal battery.

The console 14a is connected to the electronic cassette 13a by a wired or wireless method in a communicatable manner, to control the operation of the electronic cassette 13a. To be more specific, the console 14a sends the imaging condition to the electronic cassette 13a to set up a signal processing condition of the FPD (including gain of an amplifier for amplifying voltage that corresponds to the accumulated signal charge). The console 14a also performs synchronization control to synchronize accumulation and readout operation of the FPD with irradiation start and end timing of the X-ray source 10a. Furthermore, the console 14a controls turn-on and -off of the electronic cassette 13a, and puts the electronic cassette 13a into a power saving mode and a preparation mode.

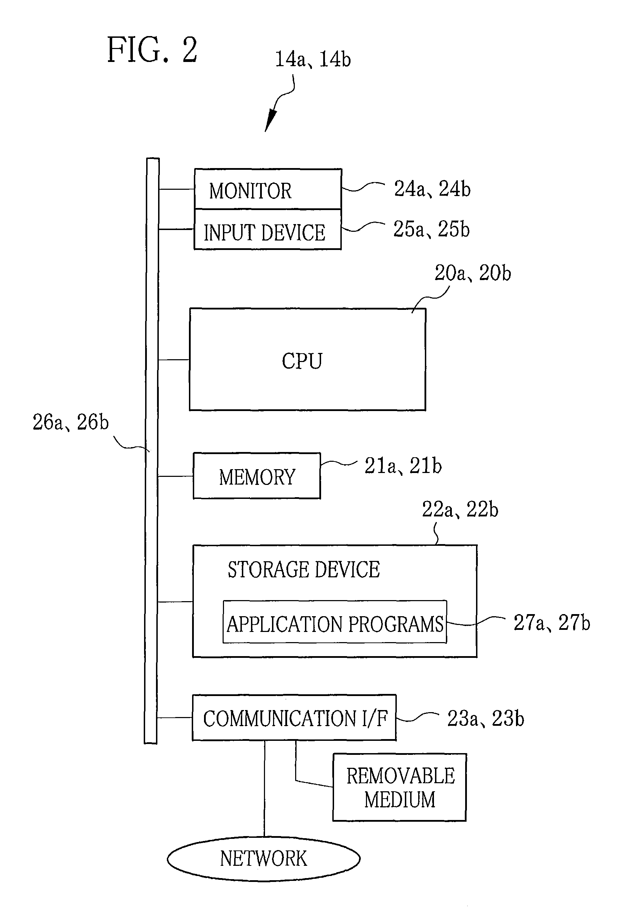

The console 14a applies various types of image processing such as an offset correction and a gain correction to X-ray image data sent from the electronic cassette 13a. The X-ray image after the image processing is displayed on a monitor 24a (see Fig. 2) of the console 14a. The X-ray image data after the image processing is written to a storage device 22a and a memory 21a (see Fig. 2) in the console 14a, or data storage such as an image server connected to the console 14a through a network.

To the console 14a, an examination order including information about sex and age of the patient, a body part to be imaged, an examination purpose, and the like is inputted from an input device 25a such as a keyboard. The examination order is displayed on the monitor 24a. The examination order is inputted from an external system e.g. HIS (hospital information system) or RIS (radiography information system) that manages patient data and examination data related to radiography, or inputted manually by the radiographic technician. The examination order includes the body part to be examined e.g. head, chest, abdomen, and the like, and an imaging direction e.g. anterior, medial, diagonal, PA (X-rays are applied from a posterior direction), and AP (X-rays are applied from an anterior direction). The radiographic technician confirms the contents of the examination order on the monitor 24a, and inputs the imaging condition corresponding to the contents of the examination order from the input device 25a through an operation screen displayed on the monitor 24a.

As shown in Fig. 2, the console 14a is composed of a computer having a CPU 20a, the memory 21a, the storage device 22a, a communication I/F 23a, the monitor 24a, and the input device 25a. These components are connected to each other via a data bus 26a. Note that, Fig. 2 has reference numerals of the console 14b of the second X-ray imaging system 2b, in addition to reference numerals of the console 14a of the first X-ray imaging system 2a.

The storage device 22a is a hard disk drive (HDD), for example. The storage device 22a stores control programs and application programs 27a. Running the application programs 27a makes the console 14a perform various functions related to radiography, such as display processing of the examination order and the X-ray image, image processing of the X-ray image, and setup of the imaging condition.

The memory 21a is a work memory used when the CPU 20a performs processing. The CPU 20a loads the control programs stored on the storage device 22a into the memory 21a, and runs the programs for centralized control of the computer. The communication I/F 23a functions as both a network interface and a medium interface for performing transmission control from/to an external device such as the RIS, the HIS, the image server, and the electronic cassette 13a, and an external storage medium such as a removable medium. The input device 25a includes a keyboard and a mouse, or a touch panel integrated with the monitor 24a.

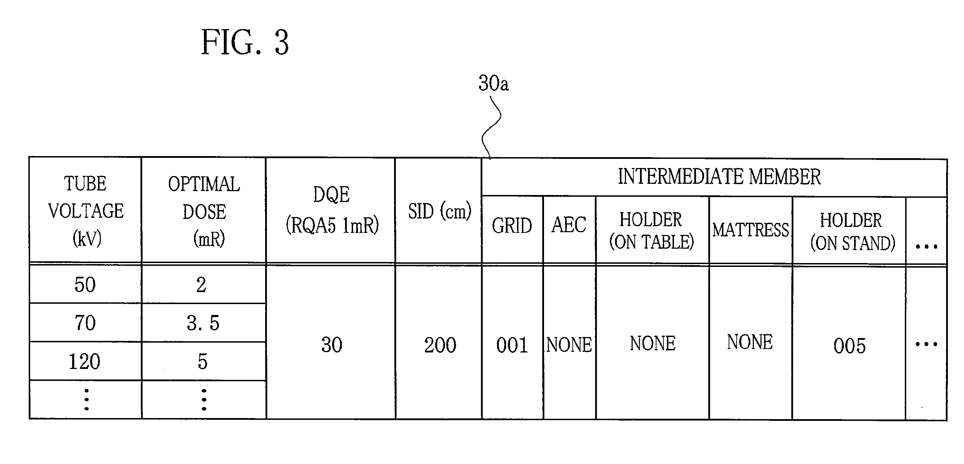

The hospital A, being a large hospital of this community area, performs a model radiation dose control. The hospital B, being an affiliated or partnership hospital of the hospital A, makes a request for sharing radiation dose information to the hospital A in order to import the model radiation dose control. In response to the request from the hospital B, the hospital A transmits specification data of the first X-ray imaging system 2a, i.e. first specification data 30a (see Fig. 3) to the hospital B. The hospital B optimizes the radiation dose control of the second X-ray imaging system 2b based on the first specification data 30a.

As shown in Fig. 3, the first specification data 30a includes items of a tube voltage (kV) of the X-ray source 10a and an optimal dose i.e. a first optimal dose (mR) at each tube voltage. The values of the tube voltage and the first optimal dose vary depending on the body part to be imaged. The optimal dose is an appropriate value of a radiation dose to be applied to the body part by which the first X-ray imaging system 2a can obtain the high-quality X-rayimage adequate for diagnosis without excessive exposure. A setup condition (tube current-time product, mAs) of the X-ray source 10a to produce the first optimal dose is derived from practical experience or obtained using a dosimeter. For example, in the case of a tube voltage of 80 kV, a source-to-image distance (SID) between the X-ray source 10a and the imaging plane of the FPD of 180 cm, and a first optimal dose of 10 mR, the tube current-time product is set at 6.4 mAs. Note that, the first optimal dose may be set more precisely in accordance with the physique of the patient e.g. thin, fat, and the like.

The first specification data 30a also includes an item of detective quantum efficiency (DQE) of the FPD of the electronic cassette 13a relative to 1 mR of X-rays of radiation quality RQA5 defined by IEC61267, an item of the SID, and an item of the type (ID) of intermediate members.

In brief, the DQE indicates sensitivity of the FPD to the X-rays or the visible light produced by the scintillator. The higher the DQE, the lower dose a radiograph requires. The intermediate member is disposed between the X-ray source 10a and the imaging plane of the FPD, and reduces the amount of the X-rays to be incident upon the FPD. The intermediate member includes a grid for removing the X-rays scattered by the patient's body, an AEC sensor for automatic exposure control by which a dose of the X-rays passed through the patient' s body is detected to stop X-ray irradiation when the dose reaches a predetermined value, a holder for holding the electronic cassette 13a on the imaging table 16a or the imaging stand 15a, a mattress put on the imaging table 16a, and the like. The intermediate member may further include a filter disposed in the X-ray source 10a for the purpose of changing the radiation quality of the X-rays by cutting a lower energy component of the X-rays. Note that, the grid is attached to the front of the electronic cassette 13a.

In this embodiment, the first optimal dose is 2, 3.5, and 5 mR at tube voltages of 50, 70, and 120 kV, respectively. The DQE is 30, and the SID is 200 cm. As for the intermediate members, a grid having an ID number "001" and a holder on the imaging stand 15a having an ID number "005" are used. Since the AEC sensor is unused, "none" is entered. The imaging table 16a has no holder and the patient lies directly over the electronic cassette 13a put on a mattress of the imaging table 16a, so "none" is entered into a holder (on table) field and a mattress field. Note that, if the AEC sensor is disposed not on a front surface of the FPD but on a back surface thereof, or the AEC sensor is out of an imaging field of the FPD, "none" is entered in an AEC sensor field because the AEC sensor does not affect an incident amount of the X-rays on the imaging plane of the FPD.

A staff member of the hospital A may transmit the first specification data 30a to a staff member of the hospital B by word of mouth over the phone, or send by mail a removable medium such as a CD-R on which the first specification data 30a is recorded. In another case, the first specification data 30a may be transmitted over a network on which both the hospitals A and B have access. In the case of transmitting the first specification data 30a on the removable medium or the network, the communication I/F 23b functions as a data acquisition section.

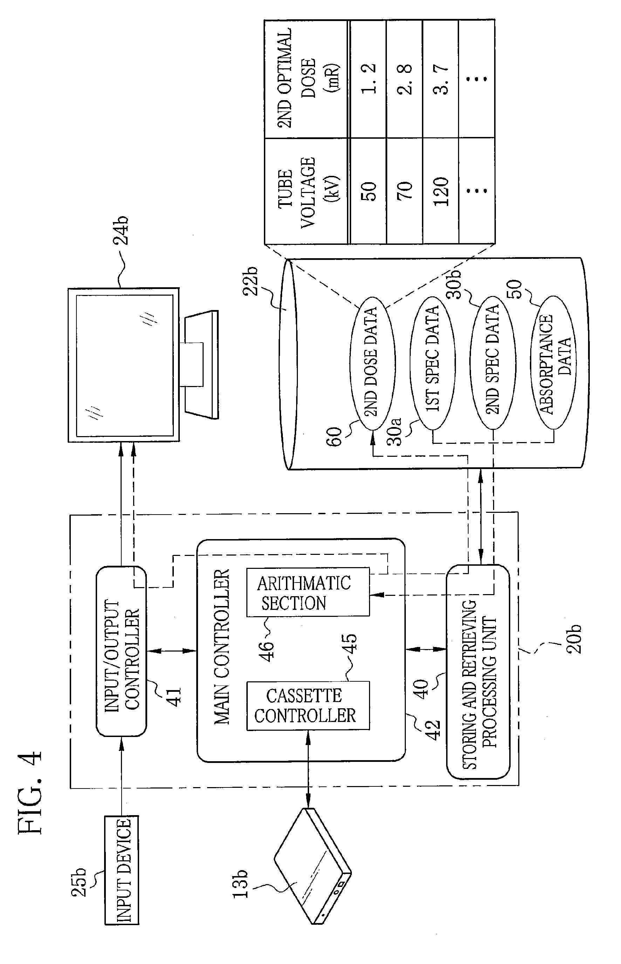

As shown in Fig. 4, by running the application programs 27b, the CPU 20b of the console 14b of the hospital B functions as a storing and retrieving processing unit 40, an input/output controller 41, and a main controller 42. The storing and retrieving processing unit 40 stores various types of data to the storage device 22b, and retrieves the data from the storage device 22b. The input/output controller 41 reads out drawing data from the storage device 22b in response to operation on the input device 25b, and outputs to the monitor 24b various operation screens of GUIs based on the read drawing data. The input/output controller 41 receives input of operation commands from the input device 25a through the operation screens. The main controller 42 includes a cassette controller 45, which controls the operation of the electronic cassette 13a, and an arithmetic section 46. The main controller 42 performs centralized control of the console 14b.

The first specification data 30a sent from the hospital A is entered into the console 14b of the hospital B manually from the input device 25b or automatically through the removable medium or the network. The storing and retrieving processing unit 40 writes the first specification data 30a to the storage device 22b.

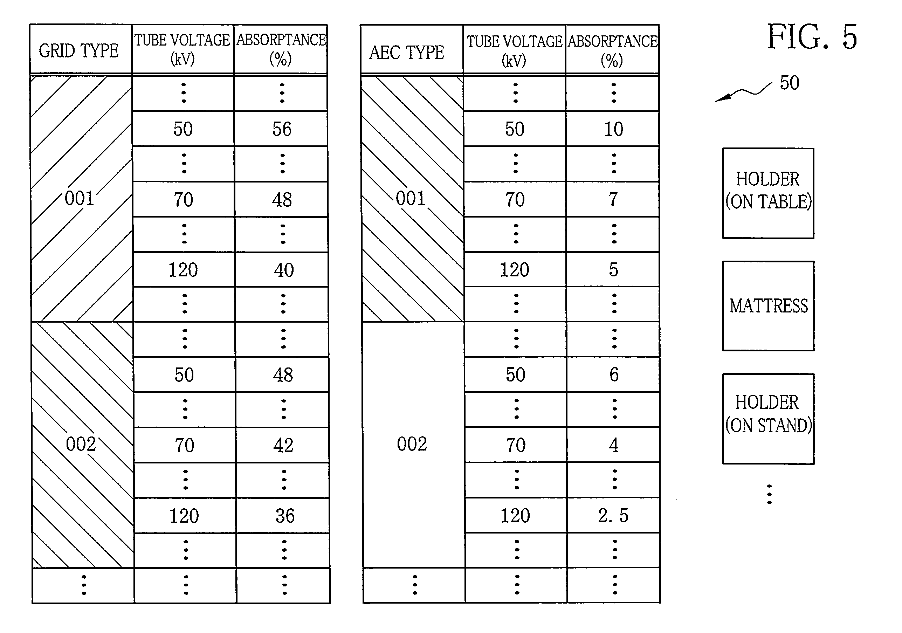

The first storage device 22b stores absorptance data 50 and second specification data 30b, in addition to the first specification data 30a. The absorptance data 50, as shown in Fig. 5, includes the type (ID) of the intermediate members including the grids and the AEC sensors, and X-ray absorptance of each type of intermediate members with respect to the tube voltage (only the grids and the AEC sensors are drawn in Fig. 5). For example, a grid of an ID number "001" has an absorptance of 40% at a tube voltage of 120 kV, an absorptance of 48 % at a tube voltage of 70 kV, and an absorptance of 56 % at a tube voltage of 50 kV. The absorptance data 50 is stored in advance on the storage device 22b at the time of shipping of the second X-ray imaging system 2b (console 14b). Whenever a manufacturer of the second X-ray imaging system 2b (console 14b) releases a new product of the intermediate member, latest absorptance data is distributed over the network or the like to update the absorptance data 50 at any time. Instead of the automatic update, the latest absorptance data of the intermediate members usable in the system 2b may be obtained from the manufacturer and inputted manually from the input device 25b.

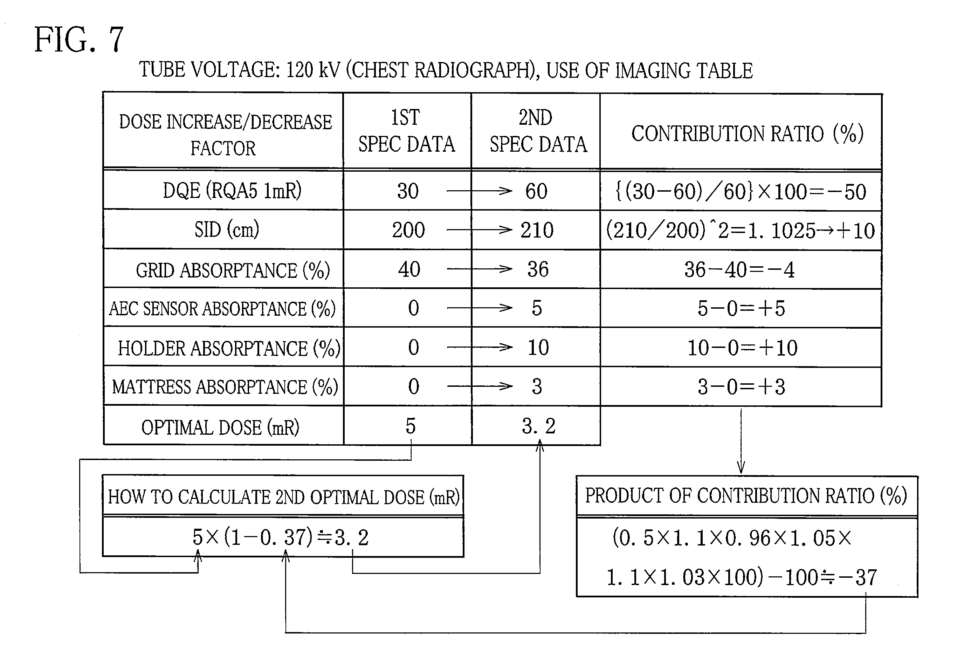

The second specification data 30b, which is specification data of the second imaging system 2b of the hospital B, corresponds to the first specification data 30a of the first X-ray imaging system 2a of the hospital A. The second specification data 30b has the same items as those of the first specification data 30a, but has different values according to the difference in the specifications between the first and second imaging systems 2a and 2b (see Fig. 7).

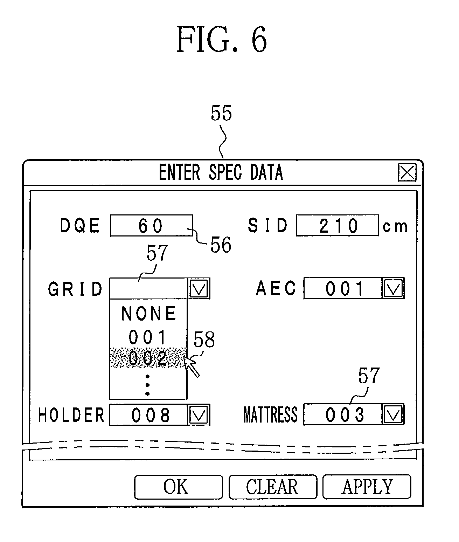

When the hospital B accepts the radiation dose information of the hospital A, the input/output controller 41 displays a data entry window 55 of Fig. 6 on the monitor 24b. The data entry window 55 has entry boxes 56 into which values of the DQE and the SID are entered, and pull-down menus 57 for choosing the ID (including "none") of the intermediate members.

In this embodiment, "60" and "210" are entered in the DQE and SID boxes, respectively. "001" is chosen as the ID of the AEC sensor. "008" is chosen as the ID of the holder (on the imaging table). "003" is chosen as the ID of the mattress. In Fig. 6, "002" is chosen as the ID of the grid, with a click on the pull-down menu 57 using a pointer 58 of the mouse.

Fig. 7 represents the first and second specification data 30a and 30b in a comparable manner. The DQE of the second imaging system 2b is twice as large as that of the first imaging system 2a. This indicates that the FPD of the electronic cassette 13b of the second imaging system 2b is superior in the X-ray or visible light sensitivity to the FPD of the first imaging system 2a. The SID of the second specification data 30b is 210 cm, while the SID of the first specification data 30a is 200 cm. This indicates that the imaging plane of the FPD of the electronic cassette 13b is another 10 cm away from the X-ray source 10b due to the addition of the AEC sensor, the holder on the imaging table 16b, and the mattress. Although the distance from the X-ray source 10a, 10b to the patient's body is the same between the first and second imaging systems 2a and 2b, the disposition of the AEC sensor, the holder, and the mattress between the body and the imaging plane of the FPD makes the imaging plane away from the X-ray source 10b by the thicknesses of the AEC sensor, the holder, and the mattress.

After the required items are entered into the data entry window 55, an OK or apply button is clicked, so the storing and retrieving processing unit 40 writes to the storage device 22b the second specification data 30b according to an entry state. Note that, the first specification data 30a may be entered manually on a similar data entry window.

The storing and retrieving processing unit 40 outputs to the arithmetic section 46 the DQE, the first optimal doses, and the SID from the first and second specification data 30a and 30b stored in the storage device 22b. Furthermore, the storing and retrieving processing unit 40 retrieves the absorptance corresponding to the type (ID) of the intermediate member from the absorptance data 50, and sends a retrieval result to the arithmetic section 46. In this embodiment, since the grid of the first specification data 30a has an ID number "001", and the grid of the second specification data 30b has an ID number "002", and the AEC sensor of the second specification data 30b has an ID number "001", values corresponding to the grids of the ID numbers "001" and "002" and values corresponding to the AEC sensor of the ID number "001" are retrieved from the absorptance data 50 and sent, as shown in hatching in Fig. 5. In addition, the absorptance of the other intermediate members, that is, the holder (on the imaging table) having an ID number "008" and the mattress having an ID number "003" is transmitted to the arithmetic section 46.

The arithmetic section 46 converts the first optimal doses of the first imaging system 2a into values (second optimal doses) corresponding to the specifications of the second imaging system 2b, based on data transmitted from the storing and retrieving processing unit 40.

As a concrete example, a radiograph is taken at a tube voltage of 120 kV (chest radiograph) with the use of the imaging table 16b. Retrieving from tables of Figs. 3 and 5, the first specification data 30a has a DQE of 30, an SID of 200 cm, a grid absorptance of 40%, no AEC sensor absorptance, no holder (on table) absorptance, no mattress absorptance, and a first optimal dose of 5 mR, as summarized in Fig. 7. On the other hand, the second specification data 30b has a DQE of 60, an SID of 210 cm, a grid absorptance of 36%, an AEC sensor absorptance of 5%, a holder (on table) absorptance of 10%, and a mattress absorptance of 3%. The DQE, the SID, and the absorptance of the intermediate members of the first and second specification data 30a and 30b are collectively called as a dose increase/decrease factor (abbreviated as factor).

The arithmetic section 46 calculates how much a dose increases or decreases by a change from the first X-ray imaging system 2a to the second X-ray imaging system 2b, in other words, a dose increase/decrease contribution ratio (abbreviated as contribution ratio) on an item-by-item basis of the factor. The contribution ratio $C_{DQE}$ of the DQE is calculated as follows:

$$C_{DQE} = \{(DQE_{old} - DQE_{new})/DQE_{new}\} \times 100$$

Wherein $DQE_{old}$ represents the DQE of the first X-ray imaging system 2a, and $DQE_{new}$ represents the DQE of the second X-ray imaging system 2b. In this embodiment, $DQE_{old}$=30 and $DQE_{new}$=60, so the DQE contribution ratio $C_{DQE}$ is calculated as follows:

$$\{(30 - 60)/60\} \times 100 = -50$$

A negative contribution ratio C indicates that the dose is decreasable by the change from the first X-ray imaging system 2a to the second X-ray imaging system 2b, while a positive contribution ratio C indicates that the dose is increased. In this embodiment, the DQE contribution ratio $C_{DQE}$ is -50%, so the dose of the second X-ray imaging system 2b is reduced into half of that of the first X-ray imaging system 2a, only with regard to the DQE. The same goes for the other items of the factor.

The contribution ratio $C_{SID}$ of the SID is calculated as follows:

$$C_{SID} = \{(SID_{new}/SID_{old})^2 - 1\} \times 100$$

Wherein $SID_{old}$ represents the SID of the first X-ray imaging system 2a, and $SID_{new}$ represents the SID of the second X-ray imaging system 2b. In this embodiment, $SID_{old}$=200 and $SID_{new}$=210, so the SID contribution ratio $C_{SID}$ is calculated as follows:

$$\{(210/200)^2 - 1\} \times 100 = +10\%$$

Note that, $SID_{new}/SID_{old}$ is squared because the dose is inversely proportional to the square of the distance.

The contribution ratio $C_{OB}$ of the intermediate member is calculated as follows:

$$C_{OB} = OB_{new} - OB_{old}$$

Wherein $OB_{old}$ represents the absorptance of the intermediate member of the first X-ray imaging system 2a, and $OB_{new}$ represents the absorptance of the intermediate member of the second X-ray imaging system 2b. "0" is substituted, when no intermediate member is used, as in the cases of the AEC sensor, the holder (on table), and the mattress of this embodiment. In this embodiment, the grid of the second X-ray imaging system 2b has a lower absorptance than that of the first X-ray imaging system 2a, so the contribution ratio of the grid becomes -4%, a negative value. The contribution ratios of the other items, i.e. the AEC sensor, the holder (on table), and the mattress become positive values, because these items are newly provided in the second X-ray imaging system 2b.

The arithmetic section 46 calculates the product $C_{pro}$ of the contribution ratios C of the individual items. This value $C_{pro}$ indicates that how much the first optimal dose is increased or decreased to be applied to the second X-ray imaging system 2b. As described above, if the $C_{pro}$ is a negative value, the optimal dose is decreased. If the $C_{pro}$ is a

positive value, the optimal dose is increased. In this embodiment, the following expression holds.

$$C_{pro} = (0.5 \times 1.1 \times 0.96 \times 1.05 \times 1.1 \times 1.03 \times 100) - 100 \cong -37\%$$

Accordingly, the first optimal dose 5 mR is reduced by 37%. In short, the second optimal dose is 63% of 5 mR.

When $P_{old}$ represents the first optimal dose, the second optimal dose $P_{new}$ is calculated as follows:

$$P_{new} = P_{old} \times \{1 + (C_{pro}/100)$$

In this embodiment, $P_{old}$=5 and $C_{pro}$=-37%, so the second optimal dose $P_{new}$ is calculated as follows:

$$5 \times (1 - 0.37) \cong 3.2$$

Therefore, the first optimal dose 5 mR is reduced into the second optimal dose 3.2 mR.

The arithmetic section 46 calculates the second optimal dose of each tube voltage (on each imaging condition such as standing position or lying position) in this manner. The storing and retrieving processing unit 40 writes the calculation results of the second optimal doses to the storage device 22b as second dose data 60, as shown in Fig. 4. In the second dose data 60, the second optimal doses are stored on the basis of the tube voltage (imaging condition). The second optimal doses may be stored on the basis of the body part to be imaged, instead. Since the body part is an essential item the radiographic technician necessarily enters from the console 14b whenever radiography is performed, storing the second optimal doses on the basis of the body part and retrieving the second optimal dose in synchronization with the entry of the body part enhance convenience.

If the imaging condition such as the body part and the tube voltage is entered (one of the body part and the tube voltage is entered because the tube voltage almost depends on the body part) from the input device 25b of the console 14b prior to taking a radiograph with the second X-ray imaging system 2b, the storing and retrieving processing unit 40 retrieves the second optimal dose from the second dose data 60 in accordance with the inputted imaging condition, and sends a retrieval result to the input/output controller 41.

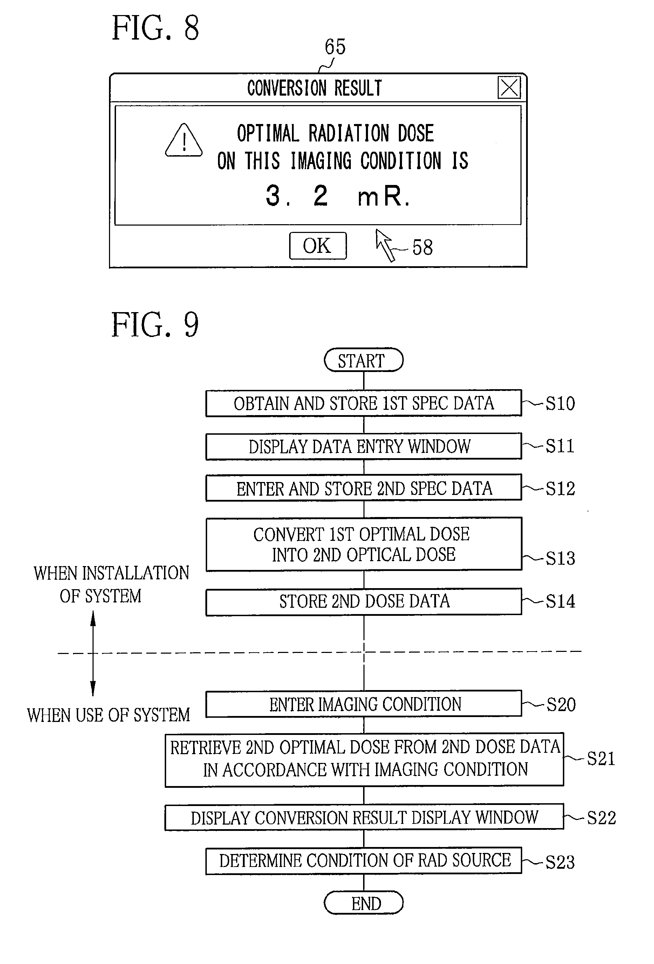

The input/output controller 41 displays on the monitor 24b a conversion result display window 65 that describes the second optimal dose as shown in Fig. 8, to inform the radiographic technician of the optimal dose suitable for the second X-ray imaging system 2b. The first optimal dose, the recommended tube voltage, and the recommended X-ray irradiation duration may be displayed together with the second optimal dose. The radiographic technician makes the setting of the imaging condition including the tube voltage and the X-ray irradiation duration from the input device 25b, such that radiography is performed with the second optimal dose seen in the conversion result display window 65. In another case, the imaging condition may be set up automatically by the main controller 42.

The operation of the above structure will be described with referring to a flowchart of Fig. 9. First in S10, the hospital B accepts the first specification data 30a of the first X-ray imaging system 2a from the hospital A at the console 14b of the second X-ray imaging system 2b, and the storing and retrieving processing unit 40 writes the first specification data 30a to the storage device 22b. Then, the data entry window 55 is displayed on the monitor 24b (S11), for the entry of the second specification data 30b of the second X-ray imaging system 2b. The second specification data 30b is written to the storage device 22b (S12).

The arithmetic section 46 converts the first optimal dose of each imaging condition into the second optimal dose based on the first and second specification data 30a and 30b and the absorptance data 50 stored in the storage device 22b (S13). Then, the conversion results are written to the storage device 22b as the second dose data 60 (S14).

When using the second X-ray imaging system 2b, upon the entry of the imaging condition such as the body part to be imaged or the tube voltage (S20), the storing and retrieving processing unit 40 retrieves from the second dose data 60 the second optimal dose corresponding to the imaging condition (S21). The retrieved second optimal dose is displayed on the conversion result display window 65 (S22). The imaging condition is determined such that radiography is performed with the optimal dose seen in the conversion result display window 65 (S23), and then the radiography is carried out.

According to the present invention, as described above, the first optimal dose is converted into the second optimal dose based on the obtained first and second specification data 30a and 30b and the absorptance data 50, and the conversion result is displayed. Therefore, it is possible to import the optimal dose of the first X-ray imaging system 2a to the second X-ray imaging system 2b with a relatively easy procedure, and equalize the image quality of the X-ray image between the first and second X-ray imaging systems 2a and 2b. If the second optimal dose is lower than the first optimal dose, radiation exposure is reduced. Also, in a case where the low-sensitive IP cassette is replaced with the high-sensitive electronic cassette, the second optimal dose becomes much lower than the first optimal dose. Thus, the present invention is effective at reducing the radiation exposure.

The absorptance data 50 of the various intermediate members facilitates precise calculation of the second optimal dose according to any change of the X-ray imaging system. The arithmetic section 46 calculates in advance the second optimal dose of each imaging condition before the radiography, and the second optimal doses are stored to the storage device 22b as the second dose data 60. Upon the entry of the imaging condition, the appropriate second optimal dose is read out from the second dose data 60. Accordingly, the radiographic technician is quickly informed of the second optimal dose after the entry of the imaging condition.

It is known that the intermediate member absorbs a low energy component of the X-rays more than a high energy component thereof, and biases the radiation quality of the X-rays so as to shift an energy peak to a high energy side (this phenomenon is called beam hardening). For this reason, if the number of the intermediate members is large, the absorptance should be calculated with correction of an energy spectrum in consideration of the beam hardening. However, the X-rays passed through the patient's body hardly contain the low energy component, and their spectra are closely analogous to those of the X-rays having only the high energy component. Thus, the optimal dose can be calculated without consideration of the beam hardening, just as with the above embodiment.

The intermediate member may be provided with a memory or a bar cord such as a RFID tag for storing its ID number. When the intermediate member is used in the X-ray imaging system, the ID number stored in the memory or the bar cord may be automatically read out to automate input of the specification data. In a like manner, input of the DQE and the SID may be automated through storing the DQE in the electronic cassette, provision of a device such as an optical sensor for automatic measurement of the SID, and the like. The automation saves time and trouble in displaying the data entry window 55 and manually inputting the specification data.

In the above embodiment, the arithmetic section 46 calculates in advance the second optimal dose of each imaging condition before the radiography, and the second optimal doses are stored to the storage device 22b as the second dose data 60. However, only input of the first and second specification data 30a and 30b is carried out before the radiography, and whenever the imaging condition is entered, the arithmetic section 46 may calculate the second optimal dose based on the imaging condition. This is suitable for a case where the size of the second dose data 60 becomes large to such an extent as to put a squeeze on the capacity of the storage device 22b due to the variety of the imaging conditions.

The hospital B shares the radiation dose information of the hospital A in the above embodiment, but the present invention is applicable to the case of changing specifications of an X-ray imaging system of a hospital (replacement of an electronic cassette or a grid, addition of an AEC sensor, and the like), the case of sharing an electronic cassette among a plurality of X-ray imaging systems in a hospital, and the like. Thus, the first and second X-ray imaging systems are not necessarily composed of separate systems, but may be substantially the same system, and more specifically may be a single system before and after changing one or more components. In short, if the present invention is applied to the case of changing the contribution ratio due to a change of the factor, the same effect as that of the above embodiment is obtained. Accordingly, a detection panel is not limited to the electronic cassette containing the FPD, but may be a film cassette, an IP cassette, or an imaging table/stand installation type of detection panel as long as its DQE is known. In this case, an ID number is allocated to each cassette, and the ID number and the optimal dose data calculated in advance may be managed in relation to each other separately from cassette to cassette having the different DQE. This facilitates quickly providing an optimal condition in response to a read of the ID number of the used cassette before taking radiography from a screen input, a bar code, an RFID tag, or the like, even in the case of using the different types of cassettes.

Other than the factors described in the above embodiment, there is aged deterioration of the X-ray source and the electronic cassette conceivable as a factor for changing the contribution ratio. Thus, the optimal dose is preferably calculated in consideration of the aged deterioration too.

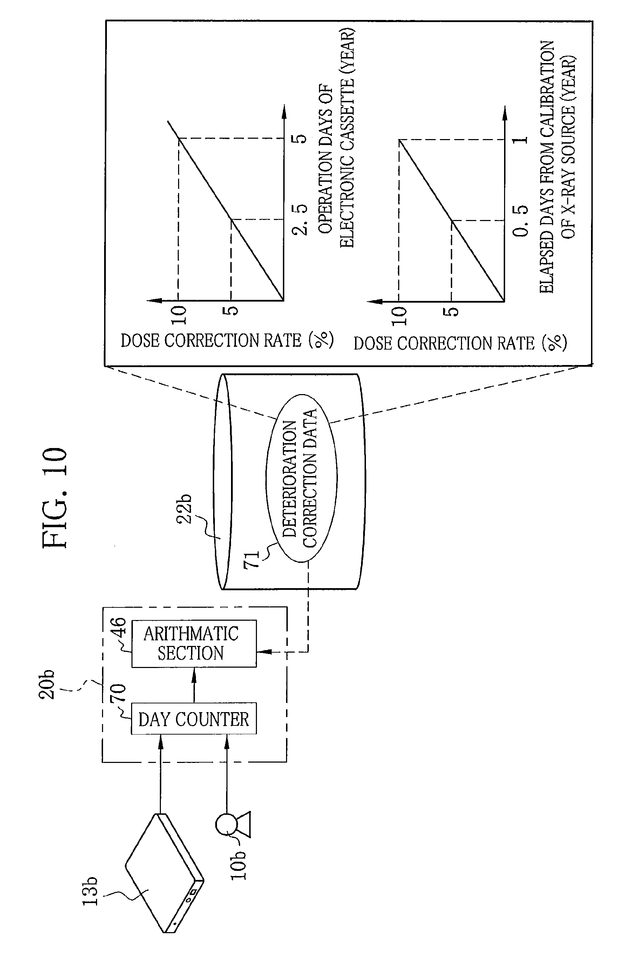

In this case, as shown in Fig. 10, the CPU 20b of the console 14b of the second X-ray imaging system 2b is provided with a day counter 70, and the storage device 22b stores deterioration correction data 71 in advance. In Fig. 10, components such as the storing and retrieving processing unit 40 and the input/output controller 41 and data such as the first and second specification data 30a and 30b are omitted. The day counter 70 counts operation days of the used electronic cassette 13b, and elapsed days from the latest calibration of the X-ray source 10b that removes an adverse effect on dose i.e. reduction of X-ray emission due to the aged deterioration. The day counter 70 outputs count data to the arithmetic section 46.

The deterioration correction data 71 represents the relation between a dose correction rate and the operation days of the electronic cassette 13b, and the relation between a dose correction rate and the elapsed days from the latest calibration of the X-ray source 10b in a data table or a functional form. The electronic cassette 13b and the X-ray source 10b are reduced in performance of X-ray detection and X-ray emission due to an extended period of use. Thus, in the deterioration correction data 71, the dose correction rate is increased with increase in the number of any of the operation days and the elapsed days. For example, the dose correction rate relative to the operation days of the electronic cassette 13b is 10% at five years of operation. The dose correction rate relative to the elapsed days from the calibration of the X-ray source 10b is 10% at one year of elapse. Adding the dose correction rate to the setup of the tube current or the X-ray irradiation duration can eliminate the deleterious effect of the aged deterioration, and carry out radiography without the deleterious effect. Note that, since the calibration of the X-ray source 10b is performed annually, the dose correction rate within one year of the elapsed days from the calibration of the X-ray source 10b is stored.

The arithmetic section 46 receives from the day counter 70 data of the operation days of the electronic cassette 13b and the elapsed days from the calibration of the X-ray source 10b. The arithmetic section 46 receives the dose correction rates corresponding to the numbers of the operation days and the elapsed days from the dose correction data 71 through the storing and retrieving processing unit 46, and adds the dose correction rates to the tube current or the X-ray irradiation duration corresponding to the second optimal dose. For example, in a case where the tube current corresponding to the second optimal dose is 100 mA, the number of operation days of the electronic cassette 13b is one year, and the calibration of the X-ray source 10b was performed six months ago (elapsed days of 0.5 year), the dose correction rate according to the number of operation days is 2%, and the dose correction rate according to the number of elapsed days is 5%. Therefore, the tube current to be actually applied to the X-ray source 10b is calculated as follows:

$$100 \times 1.02 \times 1.05 = 107.1$$

The same goes for the case of the X-ray irradiation duration, instead of the tube current. As described above, consideration of the aged deterioration, in addition to the specifications of the system, allows more accurate calculation of the optimal dose.

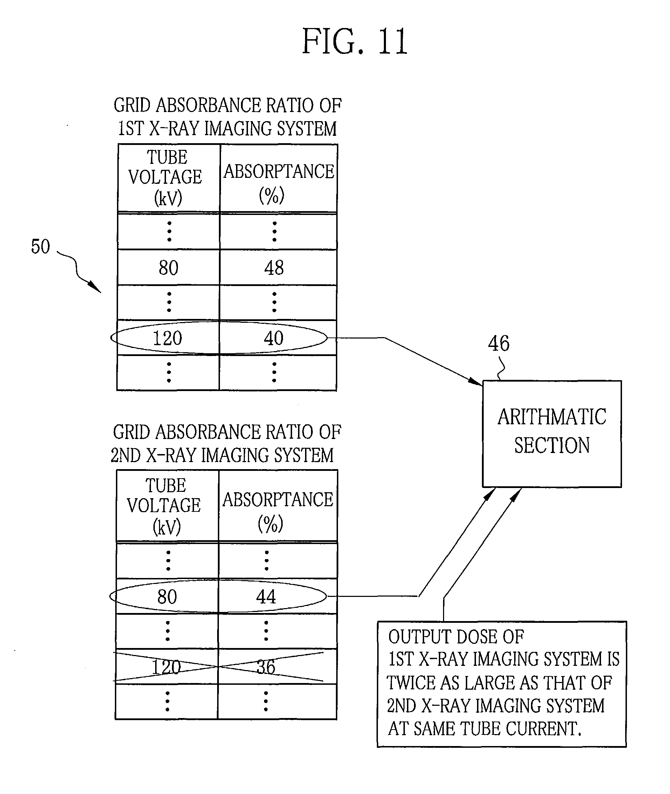

There is a case where the first and second imaging systems 2a and 2b have different output performance of the X-ray source. A case in which the tube voltage of the first X-ray imaging system 2a is 120 kV at the maximum, while that of the second X-ray imaging system 2b is 80 kV is taken as an example. In such a case, as shown in Fig. 11, the absorptance (44%) of the intermediate member e.g. the grid of the second imaging system 2b is retrieved from the absorptance data 50 referring to a row of a tube voltage of 80 kV. Using this absorptance 44% and the absorptance (40%) of the intermediate member of the first X-ray imaging system 2a, which is retrieved from a row of a tube voltage of 120 kV, the contribution ratio $C_{OB}$ is calculated. The arithmetic section 46 calculates the second optimal dose based on the contribution ratio $C_{OB}$. After that, the tube current or the X-ray irradiation duration is determined based on the second optimal dose. On the condition that the first and second X-ray imaging systems 2a and 2b have the same tube current-time product (mAs) value, if the first X-ray imaging system 2a outputs an X-ray dose of 5 mR at a tube voltage of 120 kV, and the second X-ray imaging system 2b outputs an X-ray dose of 2.5 mR at a tube voltage of 80 kV, the tube current or the X-ray irradiation duration of the second X-ray imaging system 2b is increased twice as large as that of the first X-ray imaging system 2a in order to output the same X-ray dose.

In another case, the first X-ray imaging system 2a may supply another index such as an S value, an EI value, or a REX value, which is obtained from the X-ray image data by histogram analysis. The tube current or the X-ray irradiation duration may be adjusted so as to accommodate the index of the second X-ray imaging system 2b to the index of the first X-ray imaging system 2a.

The console 14b of the hospital B takes control of the electronic cassette 13b and the like, and a computer other than the console 14b may have the function of the above arithmetic section or storage for storing the absorptance data. An external agency other than the hospitals A and B, for example, a service center of a manufacturer of the electronic cassette may have a server that plays the same role as the above console 14b of the hospital B. In this case, a CPU of the server functions as the arithmetic section, and a storage device of the server stores the absorptance data. In another case, the server has only the function of the arithmetic section, and the absorptance data may be stored in the console of each system or another data server e.g. a data server of a manufacturer of the intermediate member in a retrievable manner over a network. In this case, the first and second specification data 30a and 30b is transmitted to the server. The server calculates the second optimal dose, and transmits the second optimal dose to the client hospital. If the server of the service center deals with the calculation of the optimal dose, it is possible to take load off the console 14b of the second X-ray imaging system 2b because the console 14b does not need to have storage capacity for the absorptance data and the function of the arithmetic section.

In the above embodiment, the console 14a and the electronic cassette 13a are separate from each other. However, the console 14a may not be necessarily independent, but the electronic cassette 13a may have the function of the console 14a.

In the above embodiment, the required setup condition such as the tube current-time product is obtained from the optimal dose with the use of the practical experience or the dosimeter. However, a table representing the relation between the optimal dose and the setup condition may be prepared in advance in the storage device 22b. Note that, since the relation between the optimal dose and the setup condition varies from one type of radiation source to another, a plurality of tables corresponding to the number of types of radiation sources have to be prepared.

In this case, the first specification data 30a stores the setup condition such as the tube current-time product to be actually used by the X-ray source 10a, instead of the first optimal dose. Additionally, the first specification data 30a has an item of the type of X-ray source 10a. The second X-ray imaging system 2a extracts from the table the relation between the optimal dose and the setup condition in accordance with the type of X-ray source 10a stored in the first specification data 30a, and converts the setup condition of the first specification data 30a into the first optimal dose. A calculation procedure of the second optimal dose after this is the same as above.

To input the type of X-ray source 10b of the second X-ray imaging system 2b, the data entry window 55 of Fig. 6 is provided with a type entry box into which the radiographic technician enters the type of X-ray source 10b besides the second specification data 30b. Then, the setup condition such as the tube current-time product that corresponds to the inputted type of X-ray source 10b and the second optimal dose is extracted from the above table. The radiographic technician is notified of the extracted setup condition through the conversion result display window 65 of Fig. 8, instead of the second optimal dose. Accordingly, it is possible to easily and directly notify the radiographic technician of the setup condition such as the tube current-time product to be used by the X-ray source 10b, without measurement by the dosimeter.

The present invention is applicable to a radiation imaging system using any type of radiation, not only the X-rays but also γ-rays or the like.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. A radiation dose information sharing device for sharing radiation dose information between first and second radiation imaging systems (2a, 2b), each of said first and second radiation imaging systems (2a, 2b) including a radiation source (10a, 10b) for emitting radiation and a detection panel (13a, 13b) for detecting a radiographic image from said radiation incident on an imaging plane, said radiation dose information sharing device comprising:

    a data acquisition means (25b, 23b) for acquiring first specification data (30a) of said first radiation imaging system (2a) and second specification data (30b) of said second radiation imaging system (2b);
    a storage means (22b) for storing said first and second specification data (30a, 30b) acquired by said data acquisition means (25b, 23b); and
    an arithmetic means (46) for converting a first optimal dose into a second optimal dose suitable for said second radiation imaging system (2b) so as to equalize an image quality of an X-ray image between said first and second radiation imaging systems (2a, 2b) based on said first and second specification data (30a, 30b) read out from said storage means (22b), said first optimal dose being contained in said first specification data (30a) or calculated from said first specification data (30a), **characterised in that**
    said first and second specification data (30a, 30b) includes an item of a type of an intermediate member disposed between the radiation source and the imaging plane;
    said storage means (22b) stores absorptance data (50) representing absorptance of radiation by said intermediate member; and
    said arithmetic means (46) calculates an increase/decrease ratio of a radiation dose on a basis of said item of absorptance data read out from said storage means (22b) according to a change from said first radiation imaging system (2a) to said second radiation imaging system (2b).

2. The radiation dose information sharing device according to claim 1 further comprising a display means (24b) for displaying a conversion result of said arithmetic means (46).

3. The radiation dose information sharing device according to claim 1 or 2, wherein
    said first and second specification data (30a, 30b) further includes at least one of items of sensitivity of said detection panel (13a, 13b) and a distance between said radiation source (10a, 10b) and said imaging plane; and
    said first optimal dose varies from one imaging condition to another.

4. The radiation dose information sharing device according to claim 1, wherein said absorptance data (50) includes said absorptance of said radiation by said intermediate member, with respect to different tube voltages.

5. The radiation dose information sharing device according to claim 3, wherein said intermediate member includes at least one of a grid for removing scattered radiation, an AEC sensor for automatic exposure control, a detection panel holder provided in an imaging support (15a, 15b, 16a, 16b), a mattress put on said imaging support (15a, 15b, 16a, 16b), and an additional filter provided in said radiation source (10a, 10b).

6. The radiation dose information sharing device according to one of claims 1 to 5, wherein
said arithmetic means (46) calculates said second optimal dose of each imaging condition in advance;
said storage means (22b) stores said second optimal doses (60); and
upon input of an imaging condition, said second optimal dose corresponding to said inputted imaging condition is read out from said storage means (22b).

7. The radiation dose information sharing device according to one of claims 1 to 5, wherein whenever an imaging condition is inputted, said arithmetic means (46) calculates said second optimal dose that corresponds to said imaging condition.

8. The radiation dose information sharing device according to one of claims 1 to 7, wherein if a specification of a tube voltage of said radiation source (10a, 10b) is different between said first and second radiation imaging systems (2a, 2b), said arithmetic means (46) calculates said second optimal dose based on said absorptance data (50) of said intermediate member corresponding to the tube voltage of the second system (2b), and corrects a tube current or a radiation irradiation duration of said radiation source (10b) of said second radiation imaging system (2b) based on a ratio between said radiation dose of said first radiation imaging system (2a) and that of said second radiation imaging system (2b) in a state of having a same tube current-time product value.

9. The radiation dose information sharing device according to one of claims 1 to 8, wherein said data acquisition means includes:

   a GUI (55) displayed on display means (24b), for accepting entry of said first and second specification data (30a, 30b); and
   an input device (25b) for entering said first and second specification data (30a, 30b) through said GUI (55).

10. The radiation dose information sharing device according to one of claims 1 to 9, wherein said data acquisition means is at least one of a network interface for receiving said first and second specification data (30a, 30b) transmitted over a network and a medium interface for importing said first and second specification data (30a, 30b) from a removable medium.

11. The radiation dose information sharing device according to one of claims 1 to 10, wherein said radiation dose information sharing device is provided in a console (14b) of said second radiation imaging system (2b).

12. The radiation dose information sharing device according to one of claims 1 to 10, wherein said radiation dose information sharing device is independent from both said first and second radiation imaging systems (2a, 2b).

13. A radiation dose information sharing method for sharing radiation dose information between first and second radiation imaging systems (2a, 2b), each of said first and second radiation imaging systems (2a, 2b) including a radiation source (10a, 10b) for emitting radiation and a detection panel (13a, 13b) for detecting a radiographic image from said radiation incident on an imaging plane, said method comprising the steps of:

   acquiring first specification data (30a) of said first radiation imaging system (2a) and second specification data (30b) of said second radiation imaging system (2b);
   storing said first and second specification data (30a, 30b) to a storage means (22b); and
   reading out said first and second specification data (30a, 30b) from said storage means (22b), and converting a first optimal dose into a second optimal dose suitable for said second radiation imaging system (2b) so as to equalize an image quality of an X-ray image between said first and second radiation imaging systems (2a, 2b) based on said first and second specification data (30a, 30b), said first optimal dose being contained in said first specification data (30a) or calculated from said first specification data (30a), wherein
   said first and second specification data (30a, 30b) includes an item of a type of an intermediate member disposed between the radiation source and the imaging plane;
said storage means (22b) stores absorptance data (50) representing absorptance of radiation by said intermediate member; and
calculating an increase/decrease ratio of a radiation dose on the basis of said item of absorptance data read out from the storage means (22b) according to a change from said first radiation imaging system (2a) to said second radiation imaging system (2b).

## Patentansprüche

1. Vorrichtung zum Teilen von Strahlungsdosierungsinformation zwischen einem ersten und einem zweiten Strahlungs-Bildgebungssystem (2a, 2b), von denen jedes System (2a, 2b) eine Strahlungsquelle (10a, 10b) zum Emittieren von Strahlung und ein Detektorfeld (13a, 13b) zum Detektieren eines Strahlungsbilds aus der auf eine Abbildungsebene auftreffenden Strahlung aufweist, umfassend:

   eine Datenerfassungseinrichtung (25b, 23b) zum Erfassen erster Spezifikationsdaten (30a) des ersten Strahlungs-Bildgebungssystems (2a) und zweiter Spezifikationsdaten (30b) des zweiten Strahlungs-Bildgebungssystems (2b);
   eine Speichereinrichtung (22b) zum Speichern der ersten und der zweiten Spezifikationsdaten (30a, 30b), die von der Datenerfassungseinrichtung (25b, 23b) erfasst wurden; und
   eine Arithmetikeinrichtung (46) zum Umwandeln einer ersten optimalen Dosierung in eine zweite optimale Dosis, welche sich für das zweite Strahlungs-Bildgebungssystem (2a) eignet, um eine Bildqualität eines Röntgenbilds zwischen dem ersten und dem zweiten Strahlungs-Bildgebungssystems (2a, 2b) auszugleichen, basierend auf den ersten und den zweiten Spezifikationsdaten (30a, 30b), die aus der Speichereinrichtung (22b) ausgelesen wurden, wobei die erste optimale Dosis in den ersten Spezifikationsdaten (30a) enthalten ist oder aus den ersten Spezifikationsdaten (30a) berechnet wurde, **dadurch gekennzeichnet, dass** die ersten und die zweiten Spezifikationsdaten (30a, 30b) einen Posten eines Typs eines zwischen der Strahlungsquelle und der Abbildungsebene befindlichen Zwischenelements enthalten;
   die Speichereinrichtung (22b) Absorptionsdaten (50) speichert, die Strahlungsabsorption durch das Zwischenelement repräsentieren; und
   die Arithmetikeinrichtung (46) ein Zunahme-/Abnahmeverhältnis einer Strahlungsdosis auf einer Grundlage des Postens von Absorptionsdaten berechnet, der aus der Speichereinrichtung (22b) abhängig von einer Änderung von dem ersten Strahlungs-Bildgebungssystem (2a) zu dem zweiten Strahlungs-Bildgebungssytem (2b) ausgelesen wurde.

2. Vorrichtung nach Anspruch 1, weiterhin umfassend eine Anzeigeeinrichtung (24b) zum Anzeigen eines Umwandlungsergebnisses der Arithmetikeinrichtung (46).

3. Vorrichtung nach Anspruch 1 oder 2, bei der
   die ersten und die zweiten Spezifikationsdaten (30a, 30b) weiterhin mindestens eine der folgenden Größen enthalten: Empfindlichkeit des Detektorfelds (13a, 13b), und einen Abstand zwischen der Strahlungsquelle (10a, 10b) und der Abbildungsebene; wobei die erste optimale Dosis von einer Abbildungsbedingung zu einer anderen variiert.

4. Vorrichtung nach Anspruch 1, bei der die Absorptionsdaten (50) die Absorption der Strahlung durch das Zwischenelement enthalten, bezogen auf unterschiedliche Röhrenspannungen.

5. Vorrichtung nach Anspruch 3, bei der das Zwischenelement ein Gitter zum Beseitigen von Streustrahlung, einen AEC-Sensor für die automatische Expositionssteuerung, einen Detektorfeld-Halter in einen Bildgebungsträger (15a, 15b, 16a, 16b), eine auf den Bildgebungsträger (15a, 15b, 16a, 16b) aufgebrachte Matratze und/oder ein Zusatzfilter in der Strahlungsquelle (10a, 10b) enthält.

6. Vorrichtung nach einem der Ansprüche 1 bis 5, bei der
   die Arithmetikeinrichtung (46) die zweite optimale Dosis jeder Abbildungsbedingung vorab berechnet;
   die Speichereinrichtung (22b) die zweite optimale Dosis (60) speichert; und nach Eingabe einer Abbildungsbedingung die der eingegebenen Abbildungsbedingung entsprechende zweite optimale Dosis aus der Speichereinrichtung (22b) ausgelesen wird.

7. Vorrichtung nach einem der Ansprüche 1 bis 5, bei der immer dann wenn eine Abbildungsbedingung eingegeben wird die Arithmetikeinrichtung (46) die zweite optimale Dosis berechnet, welche der Abbildungsbedingung entspricht.

8. Vorrichtung nach einem der Ansprüche 1 bis 7, bei der, wenn eine Spezifikation einer Röhrenspannung der Strahlungsquelle (10a, 10b) zwischen dem ersten und dem zweiten Strahlungs-Bildgebungssystem (2a, 2b) abweichend ist, die Arithmetikeinrichtung (46) die zweite optimale Dosis basierend auf den Absorptionsdaten (50) des Zwischenelements entsprechend der Röhrenspannung des zweiten Systems (2b) berechnet und einen Röhrenstrom oder eine Bestrahlungsdauer der Strahlungsquelle (10b) des zweiten Strahlungs-Bildgebungssystems (2b) basierend auf einem Verhältnis zwischen der Strahlungsdosis des ersten Strahlungs-Bildgebungssystems (2a) und der des zweiten Strahlungs-Bildgebungssystems (2b) in einem Zustand gleichen Röhrenstrom produktwärts korrigiert.

9. Vorrichtung nach einem der Ansprüche 1 bis 8, bei der die Datenerfassungseinrichtung enthält:

Eine auf eine Anzeigeeinrichtung (24b) dargestellte GUI (55) zum Akzeptieren von Eingaben der ersten und der zweiten Spezifikationsdaten (30a, 30b); und
eine Eingabeeinrichtung (25b) zum Eingeben der ersten und der zweiten Spezifikationsdaten (30a, 30b) über die GUI (55).

10. Vorrichtung nach einem der Ansprüche 1 bis 9, bei der die Datenerfassungseinrichtung eine Netzwerk-Schnittstelle zum Empfangen der ersten und der zweiten Spezifikationsdaten (30a, 30b), die über ein Netzwerk übertragen wurden, und/oder eine Medium-Schnittstelle zum Importieren der ersten und der zweiten Spezifikationsdaten (30a, 30b) von einem austauschbaren Medium ist.

11. Vorrichtung nach einem der Ansprüche 1 bis 10, bei der die Strahlungsdosierungsinformations-Teilungsvorrichtung in einer Konsole (14b) des zweiten Strahlungs-Bildgebungssystems (2b) vorgesehen ist.

12. Vorrichtung nach einem der Ansprüche 1 bis 10, bei der die Strahlungsdosierungsinformations-Teilungsvorrichtung unabhängig von sowohl dem ersten als auch dem zweiten Strahlungs-Bildgebungssystem (2a, 2b) ist.

13. Verfahren zum Teilen von Strahlungsdosierungsinformation zwischen einem ersten und einem zweiten Strahlungs-Bildgebungssystem (2a, 2b), von denen jedes System (2a, 2b) eine Strahlungsquelle (10a, 10b) zum Emittieren von Strahlung und ein Detektorfeld (13a, 13b) zum Detektieren eines Strahlungsbilds aus der auf eine Abbildungsebene auftreffenden Strahlung enthält, wobei das Verfahren folgende Schritte aufweist:

Erfassen erster Spezifikationsdaten (30a) des ersten Strahlungs-Bildgebungssystems (2a) und zweiter Spezifikationsdaten (30b) des zweiten Strahlungs-Bildgebungssystems (2b);
Speichern der ersten und der zweiten Spezifikationsdaten (30a, 30b) in einer Speichereinrichtung (22b); und
Auslesen der ersten und der zweiten Spezifikationsdaten (30a, 30b) aus der Speichereinrichtung (22b) und Umwandeln einer ersten optimalen Dosis in eine zweite optimale Dosis, die sich für das zweite Strahlungs-Bildgebungssystem (2b) eignet, um dadurch eine Bildqualität eines Röntgenbilds zwischen dem ersten und dem zweiten Strahlungs-Bildgebungssystem (2a, 2b) basierend auf den ersten und den zweiten Spezifikationsdaten (30a, 30b) auszugleichen, wobei die erste optimale Dosis in den ersten Spezifikationsdaten (30a) enthalten ist oder aus den ersten Spezifikationsdaten (30a) errechnet wird, wobei
die ersten und die zweiten Spezifikationsdaten einen Posten eines Typs eines zwischen der Strahlungsquelle und der Abbildungsebene befindlichen Zwischenelements enthalten;
die Speichereinrichtung (22b) Absorptionsdaten (50) speichert, die Strahlungsabsorption durch das Zwischenelement repräsentieren; und
Berechnen eines Zunahme-/Abnahmeverhältnisses einer Strahlungsdosis auf der Grundlage des Postens von Absorptionsdaten, der aus der Speichereinrichtung (22b) abhängig von einer Änderung von dem ersten Strahlungs-Bildgebungssystem (2a) zu dem zweiten Strahlungs-Bildgebungssystems (2b) ausgelesen wurde.

**Revendications**

1. Dispositif de partage d'informations de dose de rayonnement destiné à partager des informations de dose de rayonnement entre des premier et second systèmes d'imagerie à rayonnement (2a, 2b), chacun desdits premier et second systèmes d'imagerie à rayonnement (2a, 2b) incluant une source de rayonnement (10a, 10b) destinée à émettre un rayonnement et un panneau de détection (13a, 13b) destiné à détecter une image radiographique à partir dudit rayonnement incident sur un plan d'imagerie, ledit dispositif de partage d'informations de dose de rayonnement comprenant :

un moyen d'acquisition de données (25b, 23b) destiné à acquérir des premières données de spécification (30a) dudit premier système d'imagerie à rayonnement (2a) et des secondes données de spécification (30b) dudit second système d'imagerie à rayonnement (2b) ;

un moyen de stockage (22b) destiné à stocker lesdites premières et secondes données de spécification (30a, 30b) acquises par ledit moyen d'acquisition de données (25b, 23b), et

un moyen arithmétique (46) destiné à convertir une première dose optimale en une seconde dose optimale convenant audit second système d'imagerie à rayonnement (2b) de manière à égaliser une qualité d'image d'une image radiographique entre lesdits premier et second systèmes d'imagerie à rayonnement (2a, 2b) sur la base desdites premières et secondes données de spécification (30a, 30b) lues à partir dudit moyen de stockage (22b), ladite première dose optimale étant contenue dans lesdites premières données de spécification (30a) ou calculée à partir desdites premières données de spécification (30a), **caractérisé en ce que**

lesdites premières et secondes données de spécification (30a, 30b) incluent un élément d'un type d'un membre intermédiaire disposé entre la source de rayonnement et le plan d' imagerie ;

ledit moyen de stockage (22b) stocke des données d'absorptance (50) représentant l'absorptance de rayonnement par ledit membre intermédiaire, et

ledit moyen arithmétique (46) calcule un rapport d'augmentation/diminution d'une dose de rayonnement sur une base dudit élément de données d'absorptance lues à partir dudit moyen de stockage (22b) en fonction d'un passage dudit premier système d'imagerie à rayonnement (2a) audit second système d'imagerie à rayonnement (2b).

2. Dispositif de partage d'informations de dose de rayonnement selon la revendication 1, comprenant en outre un moyen d'affichage (24b) destiné à afficher un résultat de conversion dudit moyen arithmétique (46).

3. Dispositif de partage d'informations de dose de rayonnement selon la revendication 1 ou 2, dans lequel lesdites premières et secondes données de spécification (30a, 30b) incluent en outre au moins un des éléments parmi la sensibilité dudit panneau de détection (13a, 13b) et une distance entre ladite source de rayonnement (10a, 10b) et ledit plan d'imagerie, et

ladite première dose optimale varie d'une première condition d'imagerie à une autre.

4. Dispositif de partage d'informations de dose de rayonnement selon la revendication 1, dans lequel lesdites données d'absorptance (50) incluent ladite absorptance dudit rayonnement par ledit membre intermédiaire, par rapport aux différentes tensions de tube.

5. Dispositif de partage d'informations de dose de rayonnement selon la revendication 3, dans lequel ledit membre intermédiaire inclut au moins un parmi une grille destinée à éliminer le rayonnement diffusé, un capteur AEC pour un contrôle d'exposition automatique, un support de panneau de détection prévu dans un support d'imagerie (15a, 15b, 16a, 16b), un matelas posé sur ledit support d'imagerie (15a, 15b, 16a, 16b), et un filtre supplémentaire prévu dans ladite source de rayonnement (10a, 10b).

6. Dispositif de partage d'informations de dose de rayonnement selon l'une quelconque des revendications 1 à 5, dans lequel

ledit moyen arithmétique (46) calcule ladite seconde dose optimale de chaque condition d'imagerie à l'avance ;

ledit moyen de stockage (22b) stocke lesdites secondes doses optimales (60), et

suite à la saisie d'une condition d'imagerie, ladite seconde dose optimale correspondant à ladite condition d'imagerie saisie est lue à partir dudit moyen de stockage (22b).

7. Dispositif de partage d'informations de dose de rayonnement selon l'une quelconque des revendications 1 à 5, dans lequel à chaque fois qu'une condition d'imagerie est saisie, ledit moyen arithmétique (46) calcule ladite seconde dose optimale qui correspond à ladite condition d'imagerie.

8. Dispositif de partage d'informations de dose de rayonnement selon l'une quelconque des revendications 1 à 7, dans lequel, si une spécification d'une tension de tube de ladite source de rayonnement (10a, 10b) est différente entre lesdits premier et second systèmes d'imagerie à rayonnement (2a, 2b), ledit moyen arithmétique (46) calcule ladite seconde dose optimale sur la base desdites données d'absorptance (50) dudit membre intermédiaire correspondant à la tension de tube du second système (2b), et corrige un courant de tube ou une durée d'irradiation du rayonnement de ladite source de rayonnement (10b) dudit second système d'imagerie à rayonnement (2b) sur la base d'un rapport entre ladite dose de rayonnement dudit premier système d'imagerie à rayonnement (2a) et celle dudit second système d'imagerie à rayonnement (2b) dans un état présentant une même valeur de produit courant-temps de tube.

9. Dispositif de partage d'informations de dose de rayonnement selon l'une quelconque des revendications 1 à 8, dans lequel ledit moyen d'acquisition de données inclut :

une interface utilisateur graphique (55) affichée sur un moyen d'affichage (24b), destinée à accepter une saisie desdites premières et secondes données de spécification (30a, 30b), et
un dispositif de saisie (25b) destiné à saisir lesdites premières et secondes données de spécification (30a, 30b) par le biais de ladite interface utilisateur graphique (55).

10. Dispositif de partage d'informations de dose de rayonnement selon l'une quelconque des revendications 1 à 9, dans lequel ledit moyen d'acquisition de données est au moins un parmi une interface réseau destinée à recevoir lesdites premières et secondes données de spécification (30a, 30b) transmises sur un réseau et une interface support destinée à importer lesdites premières et secondes données de spécification (30a, 30b) à partir d'un support pouvant être retiré.

11. Dispositif de partage d'informations de dose de rayonnement selon l'une quelconque des revendications 1 à 10, dans lequel ledit dispositif de partage d'informations de dose de rayonnement est prévu dans une console (14b) dudit second système d'imagerie à rayonnement (2b).

12. Dispositif de partage d'informations de dose de rayonnement selon l'une quelconque des revendications 1 à 10, dans lequel ledit dispositif de partage d'informations de dose de rayonnement est indépendant desdits premiers et seconds systèmes d'imagerie à rayonnement (2a, 2b).

13. Procédé de partage d'informations de dose de rayonnement destiné à partager des informations de dose de rayonnement entre des premier et second systèmes d'imagerie à rayonnement (2a, 2b), chacun desdits premier et second systèmes d'imagerie à rayonnement (2a, 2b) incluant une source de rayonnement (10a, 10b) destinée à émettre un rayonnement et un panneau de détection (13a, 13b) destiné à détecter une image radiographique à partir dudit rayonnement incident sur un plan d'imagerie, ledit procédé comprenant les étapes pour :

acquérir des premières données de spécification (30a) dudit premier système d'imagerie à rayonnement (2a) et des secondes données de spécification (30b) dudit second système d'imagerie à rayonnement (2b) ;
stocker lesdites premières et secondes données de spécification (30a, 30b) dans un moyen de stockage (22b), et lire lesdites premières et secondes données de spécification (30a, 30b) à partir du moyen de stockage (22b), et convertir une première dose optimale en une seconde dose optimale convenant audit second système d'imagerie à rayonnement (2b) de manière à égaliser une qualité d'image d'une image radiographique entre lesdits premier et second systèmes d'imagerie à rayonnement (2a, 2b) sur la base desdites premières et secondes données de spécification (30a, 30b), ladite première dose optimale étant contenue dans lesdites premières données de spécification (30a) ou calculée à partir desdites premières données de spécification (30a), dans lequel
lesdites premières et secondes données de spécification (30a, 30b) incluent un élément d'un type d'un membre intermédiaire disposé entre la source de rayonnement et le plan d'imagerie ;
ledit moyen de stockage (22b) stocke des données d'absorptance (50) représentant l'absorptance de rayonnement par ledit membre intermédiaire, et
calculer un rapport d'augmentation/diminution d'une dose de rayonnement sur la base dudit élément de données d'absorptance lues à partir dudit moyen de stockage (22b) en fonction d'un passage dudit premier système d'imagerie à rayonnement (2a) audit second système d'imagerie à rayonnement (2b).

# FIG. 1

HOSPITAL A

14a

10a

11a

13a

SOURCE CONTROLLER

15a

16a

12a

2a

REQUEST FOR SHARING RADIATION DOSE INFO

SEND 1ST SPEC DATA

HOSPITAL B

14b

10b

11a

13b

SOURCE CONTROLLER

15b

16b

12b

2b

EP 2 559 382 B1

# FIG. 2

14a、14b

MONITOR — 24a、24b

INPUT DEVICE — 25a、25b

20a、20b

CPU

26a、26b

MEMORY — 21a、21b

22a、22b

STORAGE DEVICE

APPLICATION PROGRAMS — 27a、27b

COMMUNICATION I/F — 23a、23b

REMOVABLE MEDIUM

NETWORK

# FIG. 3

EP 2 559 382 B1

|  TUBE VOLTAGE (kV) | OPTIMAL DOSE (mR) | DQE (RQA5 1mR) | SID (cm) | INTERMEDIATE MEMBER 30a | | | | | |
|---|---|---|---|---|---|---|---|---|---|
|  | | | | GRID | AEC | HOLDER (ON TABLE) | MATTRESS | HOLDER (ON STAND) | ... |
|  50 | 2 | 30 | 200 | 001 | NONE | NONE | NONE | 005 | ... |
|  70 | 3. 5 | | | | | | | | |
|  120 | 5 | | | | | | | | |
|  ⋮ | ⋮ | | | | | | | | |

# FIG. 4

| TUBE VOLTAGE (kV) | 2ND OPTIMAL DOSE (mR) |
|---|---|
| 50 | 1.2 |
| 70 | 2.8 |
| 120 | 3.7 |
| ⋮ | ⋮ |

# FIG. 5

| GRID TYPE | TUBE VOLTAGE (kV) | ABSORPTANCE (%) |
|---|---|---|
| | ⋮ | ⋮ |
| | 50 | 56 |
| | ⋮ | ⋮ |
| 001 | 70 | 48 |
| | ⋮ | ⋮ |
| | 120 | 40 |
| | ⋮ | ⋮ |
| | ⋮ | ⋮ |
| | 50 | 48 |
| | ⋮ | ⋮ |
| 002 | 70 | 42 |
| | ⋮ | ⋮ |
| | 120 | 36 |
| | ⋮ | ⋮ |
| ⋮ | ⋮ | ⋮ |

| AEC TYPE | TUBE VOLTAGE (kV) | ABSORPTANCE (%) |
|---|---|---|
| | ⋮ | ⋮ |
| | 50 | 10 |
| | ⋮ | ⋮ |
| 001 | 70 | 7 |
| | ⋮ | ⋮ |
| | 120 | 5 |
| | ⋮ | ⋮ |
| | ⋮ | ⋮ |
| | 50 | 6 |
| | ⋮ | ⋮ |
| 002 | 70 | 4 |
| | ⋮ | ⋮ |
| | 120 | 2. 5 |
| | ⋮ | ⋮ |
| ⋮ | ⋮ | ⋮ |

50

HOLDER (ON TABLE)

MATTRESS

HOLDER (ON STAND)

⋮

# FIG. 6

55

ENTER SPEC DATA ⊠

DQE [ 6 0 ] 56    S I D [ 2 1 0 ] cm

57

GRID [     ] ▽    AEC [ 0 0 1 ] ▽

NONE
0 0 1    58
002                        57
⋮
HOLDER [ 0 0 8 ] ▽    MATTRESS [ 0 0 3 ] ▽

[ OK ]  [ CLEAR ]  [ APPLY ]

22

# FIG. 7

TUBE VOLTAGE: 120 kV (CHEST RADIOGRAPH), USE OF IMAGING TABLE

| DOSE INCREASE/DECREASE FACTOR | 1ST SPEC DATA | 2ND SPEC DATA | CONTRIBUTION RATIO (%) |
|---|---|---|---|
| DQE (RQA5 1mR) | 30 $\longrightarrow$ | 60 | $\{(30-60)/60\}\times100=-50$ |
| SID (cm) | 200 $\longrightarrow$ | 210 | $(210/200)\hat{}2=1.1025\rightarrow+10$ |
| GRID ABSORPTANCE (%) | 40 $\longrightarrow$ | 36 | $36-40=-4$ |
| AEC SENSOR ABSORPTANCE (%) | 0 $\longrightarrow$ | 5 | $5-0=+5$ |
| HOLDER ABSORPTANCE (%) | 0 $\longrightarrow$ | 10 | $10-0=+10$ |
| MATTRESS ABSORPTANCE (%) | 0 $\longrightarrow$ | 3 | $3-0=+3$ |
| OPTIMAL DOSE (mR) | 5 | 3.2 | |

| HOW TO CALCULATE 2ND OPTIMAL DOSE (mR) |
|---|
| $5\times(1-0.37)\fallingdotseq3.2$ |

| PRODUCT OF CONTRIBUTION RATIO (%) |
|---|
| $(0.5\times1.1\times0.96\times1.05\times$ $1.1\times1.03\times100)-100\fallingdotseq-37$ |

## FIG. 8

65

```
┌─────────────────────────────────────────────────────┐
│ CONVERSION RESULT                              ⊠     │
├─────────────────────────────────────────────────────┤
│  ┌─────────────────────────────────────────────────┐│
│  │  ⚠    OPTIMAL RADIATION DOSE                     ││
│  │       ON THIS IMAGING CONDITION IS              ││
│  │                                                  ││
│  │             3. 2  mR.                            ││
│  └─────────────────────────────────────────────────┘│
│                                                      │
│              ┌──────┐    ↖                           │
│              │  OK  │      ⁀58                       │
│              └──────┘                                │
└─────────────────────────────────────────────────────┘
```

## FIG. 9

```
                    ( START )
                        │
        ┌───────────────────────────────────┐
        │  OBTAIN AND STORE 1ST SPEC DATA   │─S10
        └───────────────────────────────────┘
                        │
        ┌───────────────────────────────────┐
        │    DISPLAY DATA ENTRY WINDOW      │─S11
        └───────────────────────────────────┘
                        │
        ┌───────────────────────────────────┐
        │   ENTER AND STORE 2ND SPEC DATA   │─S12
        └───────────────────────────────────┘
                        │
        ┌───────────────────────────────────┐
        │       CONVERT 1ST OPTIMAL DOSE    │
        │       INTO 2ND OPTICAL DOSE       │─S13
        └───────────────────────────────────┘
                        │
        ┌───────────────────────────────────┐
        │       STORE 2ND DOSE DATA         │─S14
        └───────────────────────────────────┘
```

WHEN INSTALLATION OF SYSTEM

WHEN USE OF SYSTEM

```
        ┌───────────────────────────────────┐
        │       ENTER IMAGING CONDITION     │─S20
        └───────────────────────────────────┘
                        │
        ┌────────────────────────────────────────────┐
        │ RETRIEVE 2ND OPTIMAL DOSE FROM 2ND DOSE DATA│─S21
        │   IN ACCORDANCE WITH IMAGING CONDITION      │
        └────────────────────────────────────────────┘
                        │
        ┌────────────────────────────────────────────┐
        │ DISPLAY CONVERSION RESULT DISPLAY WINDOW    │─S22
        └────────────────────────────────────────────┘
                        │
        ┌───────────────────────────────────┐
        │ DETERMINE CONDITION OF RAD SOURCE │─S23
        └───────────────────────────────────┘
                        │
                    ( END )
```

# FIG. 10

EP 2 559 382 B1

# FIG. 11

GRID ABSORBANCE RATIO OF
1ST X-RAY IMAGING SYSTEM

50

| TUBE VOLTAGE (kV) | ABSORPTANCE (%) |
|---|---|
| ⋮ | ⋮ |
| 80 | 48 |
| ⋮ | ⋮ |
| 120 | 40 |
| ⋮ | ⋮ |

GRID ABSORBANCE RATIO OF
2ND X-RAY IMAGING SYSTEM

| TUBE VOLTAGE (kV) | ABSORPTANCE (%) |
|---|---|
| ⋮ | ⋮ |
| 80 | 44 |
| ⋮ | ⋮ |
| 120 | 36 |
| ⋮ | ⋮ |

46

ARITHMATIC SECTION

OUTPUT DOSE OF
1ST X-RAY IMAGING SYSTEM IS
TWICE AS LARGE AS THAT OF
2ND X-RAY IMAGING SYSTEM
AT SAME TUBE CURRENT.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010179155 A

- EP 1410761 A1